# EUROPÄISCHE PATENTANMELDUNG

(11) **EP 2 522 690 A1**
(43) Veröffentlichungstag der Anmeldung: **14.11.2012**
(21) Anmeldenummer: 11002445.2
(22) Anmeldetag: 24.03.2011
(51) Int. Cl.: C08H 8/00

(54) **Verfahren zur Herstellung von Lignin-Derivaten**

(71) Anmelder: Annikki GmbH, 8020 Graz (AT)
(72) Erfinder: Srebotnik, Ewald, Prof., 1220 Wien (AT); Ters, Thomas, Dr., 1130 Wien (AT); Fackler, Karin, Dr., 1140 Wien (AT); Messner, Kurt, Prof., 1170 Wien (AT); Ertl, Ortwin, Mag., 8010 Graz (AT)
(74) Vertreter: Schwarz, Albin

(57) **Zusammenfassung**

Verfahren zur Herstellung von Lignin-Derivaten aus technischen Ligninen durch Behandlung mit proteolytischen Enzymen (Proteasen), dadurch gekennzeichnet, dass die proteolytische Behandlung der technischen Lignine deren Molmasse signifikant vermindert.

## Beschreibung

Die wesentlichen strukturbildenden Elemente der Lignocellulose sind Cellulose, Hemicellulose und Lignin. Proteine sind zwar ebenfalls integrale Bestandteile von Lignocellulose und werden im Zuge der Biosynthese in der Pflanzenzellwand deponiert, deren biologische Funktion in der Zellwand ist jedoch weitgehend unbekannt bzw. gibt es darüber nur Mutmaßungen [1, 5].

Es ist formal möglich und in Einzelfällen bekannt, dass während dem Prozess der Lignifizierung von Pflanzenzellwänden kovalente Bindungen zwischen Lignin und Proteinen auftreten [1-5]. Es wird vermutet, dass eine derartige Vernetzung beispielsweise durch Radikalkopplung zwischen Tyrosinresten in Proteinen und phenolischen Gruppen im Lignin erfolgen kann und es gibt Belege dafür, dass Proteinablagerung und Lignifizierung in enger Beziehung zueinander stehen [4, 5]. Demnach können Proteine also nicht nur als solche in der Pflanzenzellwand vorliegen sondern auch mit anderen Zellwandkomponenten vernetzt sein. Spezifische Strukturanalysen die dies belegen könnten, stehen jedoch noch aus. Auch gibt es keinerlei Berichte über die Isolierung und Charakterisierung etwaiger Lignin-Protein-Konjugate aus Lignocellulose, die belegen würden, dass Lignin tatsächlich in einem nennenswerten Anteil aus derartigen Konjugaten besteht.

US-Patent Nr. 5,374.555 [5] offenbart ein Verfahren zur Delignifizierung von Lignocellulose mit Proteasen. In mehreren Beispielen wird dargelegt, dass die Behandlung von Lignocellulose mit Proteasen die Extraktion von Lignin aus der Lignocellulose erleichtert und verstärkt. Dieser Effekt wird darauf zurückgeführt, dass Proteine in Lignocellulose eine vernetzte Matrix ausbilden die beispielsweise an Lignin gebunden ist, weshalb die Hydrolyse dieses Proteinnetzwerks die Entfernung von Lignin aus der Lignocellulose erleichtert. Dass das extrahierte Lignin selbst signifikante Mengen an Protein enthalten könnte und dass dieses Protein einen wesentlichen Anteil an der Molmasse dieses hypothetischen Lignin-Protein-Konjugats hat, wird im US-Patent Nr. 5,374.555 [5] nicht offenbart, ebenso wenig wie eine mögliche Gewinnung von Peptiden und/oder Aminosäuren aus dem Hydrolysat.

Liang et al. [3] beschreiben ein molekularbiologisches Verfahren zur In-situ-Einführung von Peptid-Lignin-Vernetzungen in die Pflanzenzellwand um durch anschließende Behandlung der Lignocellulose mit Proteasen die Freisetzung von Polysachhariden zu verstärken. Ein möglicher Einfluss der Protease-Behandlung auf die physikalisch-chemischen Eigenschaften, beispielsweise auf die Molmasse der behandelten Peptid-Lignins-Konjugate wird nicht erwähnt.

Die vorliegende Erfindung beruht nun auf der Erkenntnis, dass Lignin-Protein-Konjugate in Lignocellulose nicht nur vorkommen, sondern überraschenderweise sogar einen beträchtlichen Anteil des Gesamtlignins ausmachen. Von den Erfindern durchgeführte Versuche haben nämlich gezeigt, dass die Behandlung mit Protease-Enyzmen dazu geeignet ist, die Molmasse von technischem Lignin signifikant zu vermindern um auf diese Weise ein Lignin-Derivat zu erlangen, welches verbesserte Werkstoffeigenschaften aufweist. In einer der Ausführungsformen der Erfindung wird daher technisches Lignin mit einem Protease-Enzym oder einem Gemisch aus verschiedenen Protease-Enzymen in einer Menge und unter Reaktionsbedingungen behandelt, die geeignet ist/sind, das/die an das technische Lignin gebundene(n) Protein(e) abzuspalten und dadurch die Molmasse des technisches Lignins signifikant zu verringern und ein deproteiniertes Lignin-Derivat zu erlangen. Die vergleichsweise geringe Molmasse der abgespaltenen Peptide und/oder Aminosäuren sowie die besonderen chemischen Eigenschaften ermöglichen in einer weiteren Ausführungsform der Erfindung deren Abtrennung vom verbleibenden Lignin-Derivat durch zusätzliche Verfahrensschritte, beispielsweise durch Ultra- und Nanofiltration, Extraktion, Fällung oder Chromatographie. In einer weiteren Ausführungsform der Erfindung werden daher die nach der Behandlung mit Protease-Enzym(en) im Hydrolysat vorhandenen Peptide und Aminosäuren vom Lignin-Derivat getrennt und zu gesonderten Produkten weiter verarbeitet.

Ein Vorteil des erfindungsgemäßen Verfahrens gegenüber herkömmlichen Verfahren zur Verminderung der Molmasse von technischem Lignin, wie z.B. thermische Spaltung und Spaltung mittels Metallkatalysatoren, ist der Reaktionsablauf unter milden Bedingungen, d.h. bei geringer Temperatur. Dadurch kann sich das Lignin nicht zersetzen und verbleibt hinsichtlich chemischer Struktur im Wesentlichen in seinem natürlichen Zustand. Technische Vorteile kleiner Lignine?

Ein besonders wesentlicher technischer Vorteil des erfindungsgemäßen Verfahrens ist die Bereitstellung von Lignin-Derivaten, die sich enzymatisch, beispielsweise durch Laccase viel besser zu Polymerisaten mit höherer Molmasse umsetzen und dadurch aktivieren lassen als nicht deproteiniertes Lignin. Wie im Folgenden ausgeführt, haben sich derartige Polymerisate ausgezeichnete Bindemittel für Holzwerkstoffe erwiesen.

Die DE 37992 C2 beschreibt ein Verfahren zur Herstellung eines Bindemittels, bei dem technisches Lignin durch Polymerisation mit Laccase und Luftsauerstoff in ein aktives Bindemittel für Holzwerkstoffe umgewandelt wird. In der DE 19700908 A1 (WO 98/31729) wird auf ähnliche Weise aus industriellem Lignin ein aktiviertes Zwischenprodukt hergestellt, welches dann in Gegenwart von Luftsauerstoff und phenoloxidierenden Enzymen, beispielsweise Laccase, mit nicht aktivierten technischen Ligninen zur Reaktion gebracht wird, wobei polymere Ligninprodukte ausgebildet werden, die eine wesentlich höhere Molmasse aufweisen als in der ohne aktiviertes Lignin durchgeführten Kontrollreaktion. Die höhere Molmasse derart aktivierter technischer Lignine hat sich als gleichbedeutend mit einer höheren Bindekraft bei Einsatz als Bindemittel erwiesen. Dabei erwies sich eine möglichst erschöpfende Oxidation des Lignins als vorteilhaft. Beispielsweise konnte in der DE 19700908 A1 (WO 98/31729) eine Steigerung der Querzugfestigkeit von Spanplatten von 6 MPa auf 11 MPa erzielt werden wenn, entsprechend einer Molmassenzunahme von 16,6%, aktiviertes Kraft-Lignin mit einer Molmasse von 6.300 g/Mol anstelle von nicht aktiviertem Kraft-Lignin mit einer Molmasse von 5.400 verwendet wurde.

Es wurde nun überraschenderweise festgestellt, dass das im erfindungsgemäßen Verfahren durch proteolytische Behandlung erlangte, deproteinierte Lignin-Derivat eine wesentlich erhöhte Reaktivität und damit Aktivierbarkeit aufweist als nicht deproteiniertes technisches Lignin. Die auf ähnliche Weise wie in DE 37992 C2 sowie DE 19700908 A1 (WO 98/31729) beschrieben durchgeführte Aktivierung eines technischen Lignins mit Laccase und Luftsauerstoff führte zu einem Anstieg der Molmasse um 40%, wogegen mit dem erfindungsgemäß deproteinierten Lignin-Derivat desselben technischen Lignins bei ansonsten identischen Reaktionsbedingungen eine schnellere Polymerisation und einen Anstieg der Molmasse um 167% erzielt werden konnte. Offenbar erhöht die Deproteinierung eines technischen Lignins in hohem Ausmaß dessen Reaktivität für eine enzymatisch katalysierte, oxidative Polymerisation. Diese Erkenntnis begründet den technischen Nutzen der vorliegenden Erfindung, denn gemäß DE 37992 C2 sowie DE 19700908 A1 (WO 98/31729) ist gerade diese Polymerisierbarkeit und die damit einhergehende Aktivierung von Lignin für die technische Eignung von Lignin-Derivaten als Bindemittel entscheidend. Gemäß DE 19700902 A1, DE 19700904 A1, DE 19700906 A1, DE 19700907 A1 und DE 19701015 A1 ist die Polymerisierbarkeit und damit einhergehende Aktivierung von Lignin außerdem für die technische Eignung von Lignin-Derivaten als Beschichtungsmittel für Papiere und andere pflanzliche Fasern, als hochreaktive Reagenzien zur Herstellung von Duroplasten, zur Herstellung von faserverstärkten Verwundwerkstoffen, sowie als Bindemittel für Holzwerkstoffe von entscheidender Bedeutung. Auch für diese Anwendungen ist ein mit der im erfindungsgemäßen Verfahren erzielbaren Verbesserung der Aktivierbarkeit von technischem Lignin einhergehender technischer Nutzen zu erwarten.

WO 98/31762, WO 98/31763 und WO 98/31764 beschreiben ebenfalls die Verwendung von technischen Ligninen oder löslichen technischen Lignin/Kohlenhydrat-Fraktionen aus lignocellulosischen Substraten von phenoloxidatierenden Enyzmen zur Polymerisation polymerisation.

Gemäß dem Stand der Technik ist davon auszugehen, dass die Verminderung der Molmasse eines technischen Lignins durch Protease-Behandlung eine mengenmäßig entsprechende Freisetzung von Peptiden und/oder Aminosäuren bewirkt. Da der Proteinanteil der erfindungsgemäßen Ligninfraktionen sehr hoch sein kann, folgt daraus, dass mit dem erfindungsgemäßen Verfahren Produkte aus technischen Ligninen hergestellt werden können, die vorteilhaft im Wesentlichen aus Peptiden und/oder Aminosäuren bestehen und nur wenig mit Lignin verunreinigt sind, was eine Gewinnung von Peptiden und/oder Aminosäuren aus nach dem erfindungsgemäßen Verfahren hergestellten Rohprodukten ökonomisch sinnvoll erscheinen lässt.

Die nachfolgenden Beispiele dienen der Illustration der vorliegenden Erfindung. In den Beispielen 1 bis 3 wird Lignin aus Stroh hergestellt und hinsichtlich Molmasse und Ligningehalt charakterisiert. Beispiel 4 illustriert darauf aufbauend den eigentlichen Kern der Erfindung, nämlich die signifikante Verminderung der Molmasse von Lignin durch Protease-Behandlung.

### Beispiel 1:

### Herstellung eines technischen Lignins im Labormaßstab.

Weizenstroh wird auf eine Partikelgröße von ca. 2 cm zerkleinert. 2,5 g zerkleinertes Weizenstroh wird in einem 500 ml Reaktionsgefäß in 200 ml einer Lösung, bestehend aus 50% Wasser und 50% Ethanol suspendiert. Die Suspension wird auf 50 °C im Wasserbad erhitzt, thermostatisiert und der pH-Wert der Suspension mit wässriger NaOH-Lösung auf einen Ausgangs-pH Wert von 13 eingestellt. Die Mischung wird bei 200 rpm, 70°C, 24h kontinuierlich gerührt.

Anschließend wurde der Feststoff abfiltriert und das klare Filtrat mit Phosphorsäure auf pH 2,0 gestellt. Der entstandene Niederschlag wurde in Dimethylformamid (DMF) gelöst und mittels präparativer Gelchromatographie an Sephadex LH60 in DMF in hoch- und niedermolekulare Fraktionen aufgetrennt. Das DMF in den einzelnen Fraktionen wurde im Hochvakuum aus den Fraktionen verdampft und der feste Rückstand im Mörser homogenisiert.

### Beispiel 2:

### Molmassenbestimmung.

Eine geeignete Probenmenge einer höhermolekularen Fraktion aus Beispiel 1 wurde in 10 mM NaOH aufgelöst und der Molmassenbestimmung mittels HPSEC an einem Agilent 1200 HPLC-System unterzogen. Das HPSEC-System bestand aus drei in Serie geschalteten Säulen von Tosoh Bioscience (G3000PW, G4000PW und G4000PW) mit 10 mM NaOH als Laufmittel. Die Kalibration erfolgte mit Polystyrensulfonat (PSS). Die Ermittlung der Molmassenverteilung der Probe erfolgte durch Auswertung der beim Absorptionsmaximum von Lignin (280 nm) aufgenommenen UV-Chromatogramme.

### Beispiel 3:

### Bestimmung des Extinktionskoeffizienten.

Exakt eingewogene Mengen Ligninfraktionen aus Beispiel 1 wurden einerseits hinsichtlich Proteingehalt (CHN-Analyse) analysiert und andererseits in DMF gelöst, gefolgt von der Ermittlung der UV-Absorption dieser Lösungen bei 280 nm im Spektralphotometer. Der Extinktionskoeffizient errechnete sich aus der Division von eingewogener Menge durch Gesamtabsorption bei 280 nm.

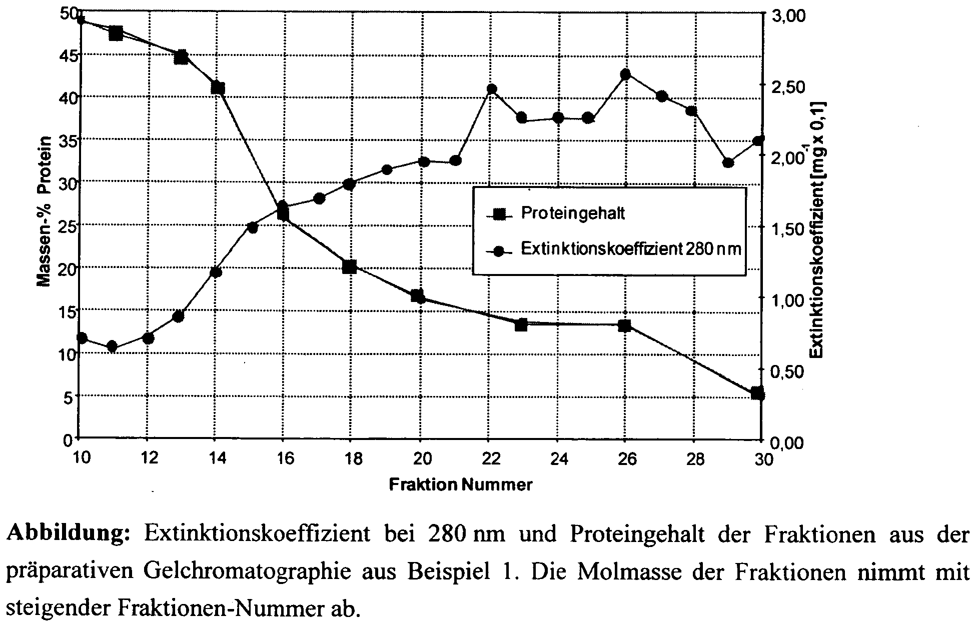

Ein typisches Ergebnis ist in der Abbildung dargestellt. Demnach erhöht sich der Extinktionskoeffizient des Lignins beim für Lignin typischen Absorptionsmaximum von 280 nm von hohen (Fraktionsnummer 10) zu niedrigen (Fraktionsnummer 30) Molmassen hin um einen Faktor 4-5 von ca. 5 mg⁻¹ auf 20-25 mg⁻¹. Wenn man gemäß dem Stand der Technik davon ausgeht, dass der Extinktionskoeffizient von reinem Lignin relativ konstant ist und die in der vorliegenden Erfindung untersuchten Fraktionen mit den höchsten Extinktionskoeffizienten (Fraktionen 21-30) aus nahezu reinem Lignin bestehen, dann müssen die hochmolekularen Fraktionen aus bis zu 80% (Fraktion 9) Nicht-Lignin bestehen. Ohne sich auf eine bestimmte Theorie festzulegen gehen die Erfinder unter Bezugnahme auf das Ergebnis aus Beispiel 4 davon aus, dass das oben genannte Nicht-Lignin hauptsächlich aus Protein besteht. Das ebenfalls in der Abbildung ersichtliche Ergebnis des aus dem Stickstoffgehalt errechneten Proteingehalts bestätigt diese Annahme: Der Proteingehalt in den Fraktionen nimmt mit abnehmender Molmasse, d.h. ansteigender Fraktionen-Nummer ebenfalls ab.

### Beispiel 4:

### Proteasebehandlung.

5 mg säuregefälltes technisches Lignin oder einer höhermolekularen Fraktionen des säuregefällten Lignins gemäß Beispiel 1 wurden in 1 ml 25 mM Tris-Puffer bei pH 8,5 gelöst und mit 0,1 mg Protease aus *Streptomyces griseus* (Sigma P5147) versetzt und bei 37°C für 1 bis 24 h gerührt. Alternativ dazu wurde Protease aus *Bacillus licheniformis* (Sigma P5380), Trpysin oder 0,1 g eines Gemisches aus den voranstehenden Proteasen mit ähnloichen Ergebnissen verwendet. Die Reaktionslösung wurde dann mit NaOH auf pH 12,0 gestellt und der HPSEC-Analyse gemäß Beispiel 2 unterzogen.

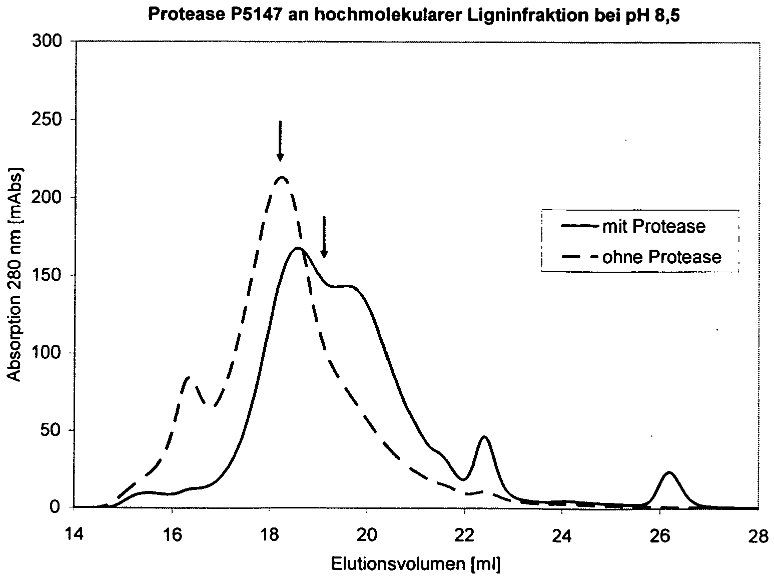

Ein typisches Ergebnis ist in der Abbildung dargestellt. Demnach resultierte die Behandlung von im Labormaßstab hergestelltem technischen Lignin mit Protease in einer signifikanten Verminderung der Molmasse von ursprünglich 16.000 Da auf ca. 6.000 Da (in der Abbildung mit Pfeilen markiert). Wie durch Vergleich der Peakflächen leicht ersichtlich ist, hat sich die Molmassenverteilung des technischen Lignins durch Protease-Behandlung stark nach rechts, d.h. zu niedrigeren Molmassen hin verschoben. Insbesondere im hochmolekularen Bereich, beispielsweise bei einem Elutionsvolumen von ca. 17 ml beträgt der Unterschied hinsichtlich Absorption bei 280 nm ein Vielfaches, was auf einen hohen Proteinanteil hinweist.

Außerdem zeigt dieses Ergebnis, dass das Protein keine lediglich in das Ligninpräparat verschleppte Verunreinigung sein kann, da ansonsten durch Protease-Behandlung keine Verschiebung der Molmasse dieses technischen Lignins zu beobachten gewesen wäre. Das UV-Spektrum der stark proteinhaltigen hochmolekularen Fraktionen war zudem typisch für Lignin. Es kann sich daher nicht lediglich um UV absorbierende Proteinstrukturen handeln, die hier detektiert wurden. Das Ergebnis zeigt viel mehr, dass Protein durch eine starke Wechselwirkung, möglicherweise kovalent an Lignin gebunden war und durch Protease-Behandlung erfolgreich abgespalten werden konnte.

Ähnliche Ergebnisse wurden auch mit anderen Ligninfraktionen und anderen Proteasen erhalten. Allgemein wurde ein umso stärkerer Einfluss von Proteasen auf die Molmasse von Lignin beobachtet, je höher die Molmasse des Lignin-Ausgangsmaterials vor der Protease-Behandlung war. Dies steht im Einklang mit dem Ergebnis aus Beispiel 3, dass der Extinktionskoeffizient des Lignins bei 280 nm mit steigender Molmasse deutlich abnahm.

### Beispiel 5:

### Polymerisation mit Laccase.

20 mg einer höhermolekularen Fraktion des technischen Lignins gemäß Beispiel 1 wurden in einer Konzentration von 4 mg/ml bei pH 8.7 mit 500 µl Protease (5 mg/ml in Wasser) gemäß Beispiel 4 versetzt. Die Kontrollreaktion erhielt 500 µl Wasser anstelle von Protease. Die Reaktionsmischungen wurden über Nacht bei 37°C inkubiert. Danach wurden nach Einstellung des pH-Wertes auf 6,0 je 0.9 ml der Reaktionslösungen mit 100 µl Laccase (1 U/ml in Wasser) versetzt und bei 30°C inkubiert. In gewissen Zeitabständen, siehe Abbildung, wurden Proben gezogen, 1:10 mit 10mM NaOH verdünnt und einer Molmassenbestimmung mittels HPSEC gemäß Beispiel 2 unterzogen.

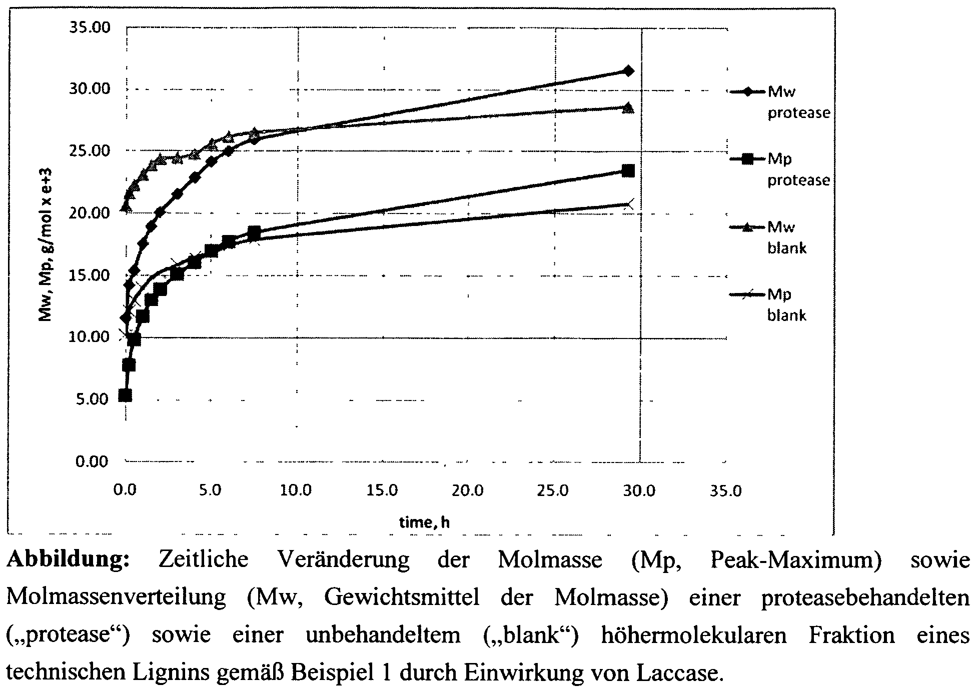

**Tabelle. Zusammenfassung der kinetischen Daten der Polymerisation einer deproteiniertem sowie unbehandeltem Ligninfraktion mittels Laccase.**

| Protease-behandelte Fraktion, Polymerisation mit 0.1 u\ml Laccase | | | | | | | |
|---|---|---|---|---|---|---|---|
| time, h | Mₙ, g/mol 10³ | ΔMn/Δt | M_{w}, g/mol 10³ | ΔMw/Δt | D | Mₚ, g/mol 10³ | ΔMp/Δt |
| 0.0 | 2.12 | | 11.57 | | 5.46 | 5.33 | |
| 0.2 | 2.36 | 1.20 | 14.23 | 13.30 | 6.03 | 7.79 | 12.32 |
| 0.5 | 2.53 | 0.58 | 15.38 | 3.83 | 6.08 | 9.82 | 6.78 |
| 1.0 | 2.70 | 0.33 | 17.57 | 4.38 | 6.52 | 11.70 | 3.75 |
| 1.5 | 2.89 | 0.39 | 18.96 | 2.78 | 6.57 | 13.03 | 2.66 |
| 2.0 | 3.00 | 0.22 | 20.09 | 2.26 | 6.70 | 13.89 | 1.72 |
| 3.0 | 3.14 | 0.14 | 21.55 | 1.46 | 6.87 | 15.09 | 1.20 |
| 4.0 | 3.27 | 0.13 | 22.90 | 1.31 | 7.01 | 16.06 | 0.94 |
| 5.0 | 3.42 | 0.16 | 24.18 | 1.32 | 7.06 | 17.01 | 0.98 |
| 6.0 | 3.52 | 0.10 | 25.01 | 0.83 | 7.10 | 17.75 | 0.74 |
| 7.5 | 3.67 | 0.10 | 25.98 | 0.65 | 7.08 | 18.51 | 0.51 |
| 29.3 | 4.25 | 0.03 | 31.59 | 0.26 | 7.43 | 23.52 | 0.23 |

| Unbehandelte Fraktion, Polymerisation mit 0.1 u\ml Laccase | | | | | | | |
|---|---|---|---|---|---|---|---|
| time, h | Mₙ, g/mol 10³ | ΔMn/Δt | M_{w}, g/mol 10³ | ΔMw/Δt | D | Mₚ, g/mol 10³ | ΔMp/Δt |
| 0.0 | 2.97 | | 20.65 | | 6.95 | 10.19 | |
| 0.2 | 3.37 | 1.97 | 21.64 | 4.95 | 6.43 | 12.13 | 9.70 |
| 0.5 | 3.58 | 0.72 | 22.29 | 2.17 | 6.23 | 13.02 | 2.97 |
| 1.0 | 3.72 | 0.28 | 23.11 | 1.64 | 6.21 | 13.99 | 1.94 |
| 1.5 | 3.88 | 0.32 | 23.87 | 1.52 | 6.15 | 14.80 | 1.62 |
| 2.0 | 3.99 | 0.21 | 24.38 | 1.02 | 6.12 | 15.25 | 0.90 |
| 3.0 | 4.07 | 0.08 | 24.48 | 0.10 | 6.03 | 15.83 | 0.58 |
| 4.0 | 4.18 | 0.11 | 24.81 | 0.33 | 5.94 | 16.46 | 0.63 |
| 5.0 | 4.23 | 0.06 | 25.64 | 0.83 | 6.06 | 16.84 | 0.38 |
| 6.0 | 4.39 | 0.15 | 26.19 | 0.55 | 5.97 | 17.39 | 0.55 |
| 7.5 | 4.52 | 0.09 | 26.55 | 0.24 | 5.88 | 17.91 | 0.35 |
| 29.3 | 5.247 | 0.03 | 28.67 | 0.10 | 5.465 | 20.81 | 0.13 |

Die in Abbildung und Tabelle dargestellten Daten belegen, dass die Polymerisation von proteasebehandeltem, d.h. deproteiniertem technischen Lignin mit viel höherer Geschwindigkeit abläuft und in einem Polymerisat mit wesentlich höherer Molmasse resultiert als in der Vergleichsreaktion mit unbehandeltem Lignin. Die Aktivierung von Lignin mit Luftsauerstoff und Laccase führte hier ohne Proteasebehandlung zu einem Anstieg der Molmasse um 40%, wogegen mit dem proteasebehandelten Lignin-Derivat desselben technischen Lignins bei ansonsten identischen Reaktionsbedingungen eine schnellere Polymerisation und einen Anstieg der Molmasse um 167% erzielt werden konnte. Ohne sich auf eine bestimmte Theorie festzulegen gehen die Erfinder davon aus, dass die Entfernung von Protein neue Reaktionszentren am Lignin hervorbringt und/oder durch Protein maskierte Reaktionszentren im Lignin freilegt, was bei Einwirkung von Oxidationsmitteln, beispielsweise Luftsauerstoff und Laccase einerseits eine wesentliche Steigerung der Polymerisationsgeschwindigkeit bewirkt und andererseits in einem Polymerisat mit wesentlich höherer Molmasse und damit einhergehender stärkerer Aktivierung resultiert.
[1] K. IIYAMA et al. (1993) Cell Wall Biosynthesis and Its Regulation. In: H.G. Jung et al. (eds.) Forage Cell Wall Structure and Digestibility, ASA-CSSA-SSSA, Madison, WI, USA.
[2] F.W. WHITMORE (1982) Phytochemistry 21, 315-316.
[3] H LIANG et al. (2008) Clean 36, 662-668.
[4] B. KELLER et al. (1989) Proc. Natl. Acad. Sci USA 86, 1529-1533.
[5] B. KELLER (1993) Plant. Physiol. 101, 1127-1130.
[6] A.R. POKORA & M.A. JOHNSON (1994) US patent No. 5,374.555.

## Patentansprüche

1. Verfahren zur Herstellung von Lignin-Derivaten aus technischem Lignin oder einer aus technischem Lignin gewonnenen Fraktion mit einem Protease-Enzym oder mit einem Gemisch aus verschiedenen Protease-Enzymen, **dadurch gekennzeichnet, dass** sich die Molmasse des technischen Lignins oder der aus technischem Lignin gewonnenen Fraktion durch Behandlung mit dem Protease-Enzym bzw. dem Gemisch aus verschiedenen Protease-Enzymen signifikant vermindert.

2. Verfahren Anspruch 1, **dadurch gekennzeichnet, dass** das technische Lignin aus lignocellulosischem Material, insbesondere Stroh, Bagasse, Energiegräser, insbesondere Elefantengras, Switchgras, und/oder Spelzen, insbesondere Deckspelzen gewonnen wird.

3. Verfahren nach Anspruch 1, **dadurch gekennzeichnet, dass** das technische Lignin ein aus einem Aufschluss von Lignocellulose gewonnenes Lignin ist.

4. Verfahren nach Anspruch 1, **dadurch gekennzeichnet, dass** das technische Lignin aus lignocellulosischem Material, insbesondere Holz, Holzfasern, Holzspäne und/oder Hackschnitzel gewonnen wird.

5. Verfahren nach einem der Ansprüche 1-4, **dadurch gekennzeichnet, dass** das aus lignocellulosischem Material gewonnene technische Lignin ein Lignosulfonat, Kraft-Lignin, Alkali-Lignin und/oder Organosolv-Lignin ist.

6. Verfahren nach einem der Ansprüche 1-5, **dadurch gekennzeichnet, dass** höhermolekulare Ligninfraktionen eingesetzt werden, die einen überdurchschnittlich hohen Proteinanteil aufweisen.

7. Verfahren nach einem der Ansprüche 1 bis 6, **dadurch gekennzeichnet, dass** die aus der Behandlung mit Protease-Enzymen entstehenden Peptide und/oder Aminosäuren vom Lignin, insbesondere durch Membranfiltration und/oder Fällung und/oder Chromatographie abgetrennt werden.

8. Verfahren nach einem der Ansprüche 1 bis 7, **dadurch gekennzeichnet, dass** die Protease-Enzyme aus der aus Protease aus *Streptomyces griseus* (Pronase, EC 3.4.24.31), Protease aus *Bacillus licheniformis* (EC 3.4.21.62), Protease aus *Staphylococcus aureus* (EC 3.4.21.19) Trypsin, Pepsin, Bromelain und Papain bestehenden Gruppe ausgewählt sind.

9. Verfahren nach einem der Ansprüche 1 bis 8, **dadurch gekennzeichnet, dass** das durch Behandlung mit Protease-Enzym(en) erlangte Lignin-Derivat eine erhöhte Reaktivität im Vergleich zum unbehandelten technischen Lignin aufweist.

10. Verfahren nach einem der Ansprüche 1 bis 9, **dadurch gekennzeichnet, dass** das durch Behandlung mit Protease-Enzym(en) erlangte Lignin-Derivat eine hinsichtlich Polymerisationsgeschwindigkeit und/oder Polymerisationsgrad verbesserte Polymerisierbarkeit, insbesondere bei oxidativer Polymerisation mit phenoloxidierenden Enzymen, beispielsweise Laccase, aufweist.
